# EUROPEAN PATENT SPECIFICATION

(11) **EP 2 427 222 B1**
(45) Date of publication and mention of the grant of the patent: **19.06.2013**
(21) Application number: 10734590.2
(22) Date of filing: 03.05.2010
(51) Int. Cl.: A61L 15/18, A61L 15/20, A61L 15/42, A61L 15/46

(54) **HYGIENE ARTICLE HAVING CALCIUM SUGAR PHOSPHATE**
HYGIENEARTIKEL MIT CALCIUMZUCKERPHOSPHAT
ARTICLE D'HYGIÈNE COMPORTANT UN PHOSPHATE DE SUCRE DE CALCIUM

(30) Priority: 05.05.2009 US 435633
(43) Date of publication of application: 14.03.2012
(73) Proprietor: The Procter & Gamble Company, Cincinnati, OH 45202 (US)
(72) Inventor: WANG, Fancheng, Blue Ash Ohio 45242 (US); NEMETH, Kimberly, Ann, Mason Ohio 45040 (US); OSBORN, Thomas, Ward, III, Cincinnati Ohio 45220 (US)
(74) Representative: Kremer, Véronique Marie Joséphine
(86) International application number: PCT/US2010/033326
(87) International publication number: WO 2010/129444

(56) References cited:
- WO-A1-2009/003073
- WO-A2-2009/149264
- US-A1- 2003 158 156
- US-A1- 2008 166 307

## Description

### FIELD OF THE INVENTION

The present invention relates to hygiene articles having one or more calcium sugar phosphates, wherein the calcium sugar phosphates reduce the production of Toxic Shock Syndrome Toxic-1.

### BACKGROUND OF THE INVENTION

*Staphylococcus aureus (S. aureus)* is a bacterium that commonly colonizes human skin and mucous membranes. It causes disease in humans through the production of toxic proteins. One such disease is Toxic Shock Syndrome (TSS), caused by Toxic Shock Syndrome Toxin-1 (TSST-1) and other similar toxins. When absorbed into the blood stream, TSST-1 may lead to TSS in non-immune humans. It has been thought that TSS is associated with growth of S. *aureus* in the presence of tampons, such as those used in nasal packing or as catamenial devices. *S. aureus* is found in the vagina of approximately 10% of healthy women of menstrual age. Approximately 1% of the *S. aureus* isolates from the vagina are found to produce TSST-1.

Symptoms of TSS generally include fever (>102°F), diarrhea, vomiting, a sunburn-like rash, hypotension, and multiple organ failure. Multiple organ failure occurs in approximately 6% of those who contract the disease. As *S. aureus* grows and multiplies, it can produce TSST-1. In some cases, only after entering the bloodstream does TSST-1 toxin act systemically and produce the symptoms attributed to TSS.

When *S. aureus* is present in an area of the human body that harbors a normal microbial population such as the vagina, it may be difficult to eradicate the *S. aureus* bacteria without harming members of the normal microbial flora required for a healthy vagina. Typically, antibiotics that kill *S. aureus* are not a viable option for use in feminine articles. This is because of their effect on the normal vaginal microbial flora and their tendency to stimulate toxin production in the *S. aureus* that are not killed. An alternative to eradication is technology designed to prevent or substantially reduce the bacteria's ability to produce toxins.

There have been attempts in the prior art to reduce or eliminate pathogenic microorganisms and menstrually occurring TSS by incorporating into a feminine article one or more biostatic, biocidal, or detoxifying compounds. For example, L-ascorbic acid has been applied to a tampon to detoxify toxin found in the vagina. Other examples have incorporated into tampons monoesters and diesters of polyhydric aliphatic alcohols, such as glycerol monolaurate, as potential biocidal compounds. Still further examples have introduced to tampons non-ionic surfactants, such as alkyl ethers, alkyl amines, and alkyl amides as detoxifying compounds.

US2008/166307 discloses oral care compositions comprising a calcium ion donor, i.e. calcium salts, such as, e.g., calcium glycerophosphate, calcium phosphate, calcium gluconate, calcium carbonate, calcium chloride, calcium sulfate, calcium oxide or mixtures thereof in order to improve oral hygiene.

US2003/158156 discloses a method of inhibiting the production of TSST-1 from Gram positive bacteria comprising exposing the Gram positive bacteria to an effective amount of a first active ingredient, i.e. hexachlorophene, benzylparaben, benzyl salicylate, benzophenone-6,-7,-8,-9,-10,-12,-1,-2,-3, etc.

WO2009/149264 discloses antibacterial compositions comprising calcium sugar phosphate selected from calcium glycerophosphate that is useful for inhibiting bacterial growth in cosmetics and pharmaceutical uses, such as on a carrier which is suitable for topical application to skin, that is to say for hygiene articles. The calcium sugar phosphate reduces *S. aureus.*

Despite the above mentioned attempts, there continues to be a need for compounds that will effectively inhibit the production of TSST-1, without impacting the normal vaginal flora. Further, it is desirable that the TSST-1 reducing compounds are non-harmful to *S. aureus.*

### SUMMARY OF THE INVENTION

A hygiene article is provided that comprises calcium sugar phosphate. The calcium sugar phosphate reduces *S. aureus* TSST-1 production as measured by ELISA, as compared to TSST-1 production by *S. aureus* to which no calcium sugar phosphate has been added.

A method of producing a hygiene article having a calcium sugar phosphate is provided. The method comprises the steps providing a hygiene article, providing a calcium sugar phosphate, and adding the calcium sugar phosphate to the hygiene article.

A hygiene article is provided that comprises a combination of a sugar phosphate and calcium. The combination of a sugar phosphate and calcium reduces *S. aureus* TSST-1 production as measured by ELISA, as compared to TSST-1 production by *S. aureus* to which no combination of a sugar phosphate and calcium has been added.

A method of producing a hygiene article having a calcium sugar phosphate is provided. The method comprises the steps of providing a calcium sugar phosphate, providing a fiber, adding the calcium sugar phosphate to the fiber, and forming a hygiene article from the fiber.

### DETAILED DESCRIPTION OF THE INVENTION

The present invention is directed to hygiene articles comprising one or more calcium sugar phosphates. The vaginal environment is host to a variety of microorganisms, some of which are potential pathogens. One potential pathogen is the bacteria *Staphylococcus aureus (S. aureus). S. aureus* can produce Toxic Shock Syndrome Toxin-1 (TSST-1), which has been associated with the onset of Toxic Shock Syndrome. The present invention, of hygiene articles comprising calcium sugar phosphates, reduces the amount of TSST-1 produced by *S. aureus* without significantly affecting the growth of *S. aureus.*

As used herein, the term "hygiene article" refers to articles: (1) that absorb, block, or contain bodily fluids, such as urine, blood, or menses, and which may be placed within, against or in proximity to the body of a user; and (2) where during the hygiene article's use it would be beneficial to reduce or prevent the generation of toxins from Gram positive bacteria, such as *S. aureus.* Suitable articles can include medical articles, feminine articles, adult incontinence products, and diapers.

As used herein, the term "medical articles" includes absorbent articles, such as absorbent articles intended for medical, dental, surgical or nasal use, such as bandages, surgical wound dressings, sponges, and nasal packing.

As used herein, the term "feminine article" includes absorbent articles and non-absorbent articles intended to be worn in or near the vagina. Absorbent feminine articles can be worn by women for menstrual or light incontinence control, for example tampons, sanitary napkins, intralabial devices, incontinence articles, and liners. Non-absorbent feminine articles can include pessaries for the treatment of vaginal prolapse or incontinence, cervical caps, contraceptive sponges, menstrual cups, or contraceptive diaphragms.

As used herein, the term "absorbent article" refers to devices comprising one or more absorbent materials that absorb or contain a bodily fluid, such as urine, blood, or menses. A typical absorbent article can be placed within, against or in proximity to the body of the wearer to absorb and contain various bodily fluids.

The term "absorbent material" as used herein can be constructed from a wide variety of materials commonly used in absorbent articles. Such materials include, but are not limited to synthetic fibers, natural fibers, or combinations thereof, which may be used to produce a fiber matrix. The natural fibers may include, but are not limited to, cotton, wood pulp, flax, hemp, and rayon, such as GALAXY Rayon (a tri-lobed rayon structure) available as 6140 Rayon; or SARILLE L rayon (a round fiber rayon), both available from Kelheim Fibers GmbH of Kelheim, Germany, cotton, wood pulp, flax, and hemp. The synthetic fibers can include, but are not limited to, fibers such as polyester, polyolefin, nylon, polypropylene, polyethylene, polyacrylic, vinyl polyacetate, polylactic acid, polyhydroxyalkonate, polyglycolic acid, polyacrylate, styrene block copolymers, cellulose acetate, or bicomponent fibers, such as bicomponent polyethylene and polypropylene fibers. Additional absorbent material include materials such as, peat moss, capillary channel fibers (such as those disclosed in U.S. Patent No. 5,356,405), high capacity fibers (such as those disclosed in U.S. Patent No. 4,044,766), superabsorbent polymers or absorbent gelling materials (such as those disclosed in U.S. Patent No. 5,830,543), may be incorporated into the tampon. Further, absorbent materials may comprise absorbent foams. Absorbent foams may include materials such as polyurethane, melamine-formaldehyde, polyvinyl alcohol-formaldehyde, or cellulose. Another example of absorbent foams that can be used in the present invention are foams that include polymeric foam materials that result from the polymerization of certain water-in-oil emulsions having therein a relatively high ratio of water phase to oil phase. Emulsions of this type, which have these relatively high water to oil phase ratios are known in the art as high internal phase emulsions ("HIPEs" or "HIPE" emulsions). The polymeric foam materials that result from the polymerization of such emulsions are referred to as "HIPE foams." Examples of HIPE foams are found in U.S. Pat. No's 5,260,345; 5,387,207; 5,817,704; 5,550,167; 5,827,909; 6,365,642; 6,369,121; 6,525,106; 6,362,244.

As used herein, the term "tampon" refers to any type of absorbent article that is inserted into the vaginal canal for the absorption of fluid therefrom. Typically, tampons are constructed from an absorbent material, which may be in the form of a pledget that has been compressed into a vaginally insertable shape.

As used herein, the term "pledget" refers to a construction of absorbent material prior to the compression of such construction into a tampon. A pledget can have a variety of shapes, including but not limited to, oval, round, chevron, square, rectangular, trapezoidal, and the like. To produce a tampon, a pledget may be compressed into a generally cylindrical configuration in the radial direction, axially along the longitudinal axis or in both the radial and axial directions.

A pledget may include one or more overwraps. The overwrap can be any suitable material, such as, for example, rayon, cotton, bicomponent fibers, polyethylene, polypropylene, polylactic acid, polyhydroxyalkonate, polyglycolic acid, styrene block copolymers, other suitable natural or synthetic fibers known in the art, and mixtures thereof. In certain embodiments, a pledget can comprise an overwrap material that substantially encloses the pledget. In certain embodiments, a pledget can include an overwrap material that extends beyond the withdrawal end and forms a finger cover or absorbent skirt.

A pledget can additionally include a withdrawal member. The withdrawal member can be any suitable configuration, such as one or more cords, strings, finger covers, ribbons, an extension of a material of the device, or combinations thereof. The withdrawal member can be made of any suitable material, such as cotton or rayon. The withdrawal member can optionally be provided with a secondary absorbent member, such as a mass of secondary absorbent material attached to the withdrawal member proximate the withdrawal end of the pledget. Examples of secondary absorbent members that may be used are described in U.S. Patent No. 6,258,075.

As used herein the term substantially "non-lethal" with regard to S. *aureus* means the cell density of *S. aureus* in the test fluid containing a calcium sugar phosphate and *S. aureus,* grown using the Shake Flask Method, is not reduced by more than a factor of about 10² CFU/ml (2 log) of test fluid relative to the *S. aureus* grown in the absence of the calcium sugar phosphate using the Shake Flask Method.

As used herein the term substantially "lethal" with regard to *S. aureus* means the cell density of test fluid containing a calcium sugar phosphate and *S. aureus* grown using the Shake Flask Method, is reduced by a factor of at least about 10³ CFU/mL (3 log) relative to test fluid containing *S. aureus* grown using the Shake Flask Method, but not containing a calcium sugar phosphate.

As used herein, the term "stable" means the calcium sugar phosphate can have TSST-1 reducing capability when exposed to environmental conditions, such as water vapor (humidity) or temperatures above or below about room temperature (temperatures above or below about 25°C) during manufacture or storage.

As used herein the term "fugitive" means the calcium sugar phosphate is capable of moving through the fiber matrix of the absorbent material of an absorbent article.

As used herein, the term "bound" means less than about 10% of the calcium added to an absorbent article is removed by soaking the absorbent article over an 8 hour period at 37°C in three times the syngyna capacity of sterile physiologic saline solution. The syngyna capacity is determined by the syngyna test (U.S. FDA 21 CFR 801.430, Revised as of April 1, 2006). The percent of "bound" calcium is calculated as (calcium present in the article - calcium in solution) divided by calcium present in the article.

As used herein, the term "partially bound" means less than about 50% of the calcium added to the absorbent article is removed by soaking the article over an 8 hour period at 37°C in three times the syngyna capacity of sterile physiologic saline solution.

As used herein, the term "substantially bound" means less than about 25% of the calcium added to the absorbent article is removed by soaking the article over an 8 hour period at 37°C in three times the syngyna capacity of sterile physiologic saline solution.

In certain embodiments, calcium sugar phosphates are calcium salts of phosphate esters of sugars or sugar alcohols, having from about 3 carbon atoms to about 7 carbon atoms (C₃ to C₇). Examples of calcium sugar phosphates include calcium dihydroxyacetone phosphates; calcium glycerophosphates; calcium hexosephosphates, such as calcium fructosephosphates, calcium galactosephosphates, calcium glucosephosphates, calcium hexosediphosphate, and calcium mannosephosphates; calcium inositol phosphates, such as calcium inositol 1,4,5 triphosphate; calcium mannitol phosphates; and calcium pentosephosphates.

In certain embodiments, calcium sugar phosphates may have the following chemical structure:

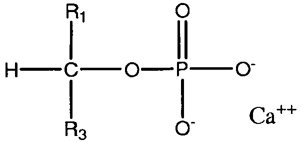

Wherein:
R₁, R₃ = H; (CHOH)ₙCH₂OH; (CHOH)ₙC(O)R₄; (CHOH)ₙ CHO-PO₃²⁻
R₄ = H; (CHOH)ₘ CH₂OH; (CHOH)ₘ CHO-PO₃²⁻
n =0- 5
m = 0-4
n+m =0-4

Examples of calcium sugar phosphates include:

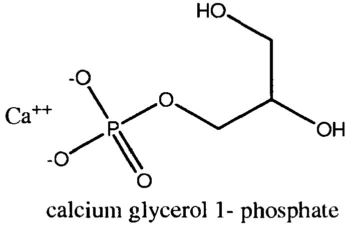

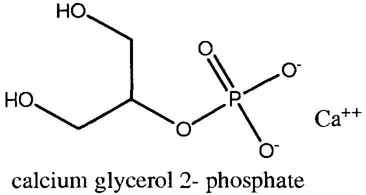

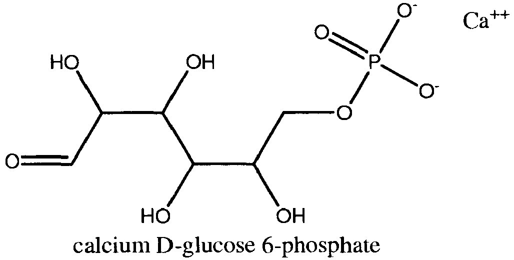

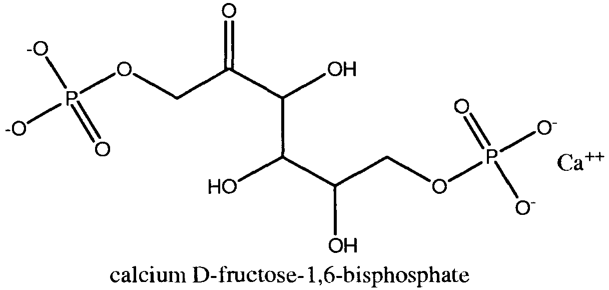

In certain embodiments, a hygiene article can include a unit dose of one or more calcium sugar phosphates. In certain embodiments, a unit dose is an amount of one or more calcium sugar phosphates that are added to a hygiene article to provide a desired reduction in TSST-1 (as compared to TSST-1 produced by *S. aureus* to which no calcium sugar phosphate was added). In certain embodiments, the unit dose of calcium sugar phosphate is an amount suitable to provide the hygiene article with greater than 0.009 millimoles of calcium, greater than 0.01 millimoles calcium, greater than 0.02 millimoles calcium, greater than 0.04 millimoles calcium, greater than 0.06 millimoles calcium, greater than 0.08 millimoles calcium, greater than 0.1 millimoles calcium, greater than 0.5 millimoles calcium, greater than 1 millimoles calcium, greater than 2 millimoles calcium, greater than 3 millimoles calcium, greater than 4 millimoles calcium, greater than 5 millimoles calcium, greater than 6 millimoles calcium, greater than 7 millimoles calcium, greater than 8 millimoles calcium, greater than 8 millimoles calcium, greater than 9 millimoles calcium, greater than 10 millimoles calcium or more. In certain embodiments, less than substantially all of the calcium sugar phosphate that is added to the article is available to reduce the production of TSST-1, for example when some of the calcium sugar phosphate is retained within the hygiene article during use. The production of TSST-1 by *S. aureus* to which one or more calcium sugar phosphates have been added, can be reduced by any suitable amount, such as by at least about 70%, 80%, 90%, 95%, or more, as compared to TSST-1 production by *S. aureus* to which no calcium sugar phosphate has been added. In certain embodiments, the amount of TSST-1 produced by *S. aureus* can be measured using the Enzyme-Linked ImmunoSorbent Assay (ELISA) technique.

In addition, the one or more calcium sugar phosphates can be substantially non-lethal to *S. aureus,* as demonstrated when *S. aureus* is grown using the shake flask method, and the colony forming units (cfu) determined.

In certain embodiments, the calcium sugar phosphate can have a solubility that provides a Ca concentration of at least about 40 millimoles/L of water at 25°C, such as, for example at least about 45 millimoles/L of water at 25°C, at least about 50 millimoles/L of water at 25°C, at least about 60 millimoles/L of water at 25°C, at least about 70 millimoles/L of water at 25°C, at least about 80 millimoles/L of water at 25°C, or more.

In certain embodiments, the TSST-1 reducing properties of calcium sugar phosphate will not be substantially diminished as a result of the tampon making process, tampon packaging, or tampon storage. For example, the calcium sugar phosphate can retain its ability to inhibit the production of TSST-1 following a tampon making process or when stored in conditions, such as high or low temperatures related to the commercial manufacture and sale. In certain embodiments, the calcium sugar phosphate can retain its ability to inhibit the production of TSST-1 for any suitable time at any suitable temperature, such as at about 100°C for about 3 hours, from -30°C to 65°C for about 24 hours, from about 0°C to about 50°C, or any other suitable temperature.

In certain embodiments, the calcium sugar phosphate can resist acquisition of moisture in a humid environment. Absorbent articles, such as tampons can be exposed to conditions where the relative humidity can exceed 80%, for example during commercial storage or in the bathroom during a shower. Some salts, such as calcium chloride, magnesium chloride, and zinc chloride, are deliquescent substances that can have a strong affinity for moisture and can absorb a relatively large amount of fluid from the atmosphere. The use of a deliquescent substance in a tampon can result in moisture being drawn into the tampon, such that the tampon can expand, causing difficulties with an applicator during insertion into the body or expulsion therefrom.

A unit dose of calcium sugar phosphate can be added to a hygiene article by any suitable process or at any step in the hygiene article manufacturing or packaging process. The calcium sugar phosphate can be added to one or more portions of a hygiene article. One such example can be a tampon having the calcium sugar phosphate added into or on the primary absorbent member, the overwrap, the secondary absorbent member, the withdrawal member, or combinations thereof. In certain embodiments, the calcium sugar phosphates can also be added on, within or throughout one or more portions of the tampon.

In certain embodiments, a calcium sugar phosphate can be added to a hygiene article or a portion thereof, such as fibers using one or more pharmaceutically acceptable and compatible carrier materials. Some suitable examples of carrier materials include aqueous solutions, gels, suspensions, foams, lotions, balms, salves, ointments, boluses, suppositories, or combinations thereof. In certain embodiments, it is also possible to add the calcium sugar phosphate as a powder.

In certain embodiments, a calcium sugar phosphate can be added to the fiber forming the absorbent material. The calcium sugar phosphate can be added directly into the fiber during manufacturing of the fiber. In certain embodiments, such as, for example when using polyethylene fibers, polypropylene fibers, polyethylene terephthalate fibers, conjugate fibers, bicomponent fibers, rayon fibers, or any other suitable synthetic fibers, the calcium sugar phosphate can be added to the melt prior to the formation of the fibers. As the resulting fibers cool, the calcium sugar phosphate can migrate to the surface of the fiber. In certain embodiments, a unit dose of calcium sugar phosphate can be added such that the amount of calcium that migrates to the surface of the fiber is sufficient to reduce TSST-1 production. The concentration of the calcium sugar phosphate added to the polymer melt can be any suitable concentration, for example between 10% and 30% or between 15% and 25% of the fiber weight.

In certain embodiments, a calcium sugar phosphate can be added to a fiber during a hygiene article forming process, such as the tampon making process prior to forming the pledget, for example, in the fiber washing and drying steps or when a fiber finishing agent is added to facilitate fiber processing. In addition, a calcium sugar phosphate can be added to a fiber after the pledget is made. For example, a calcium sugar phosphate can be added to one or more layers of a pledget prior to compression by exposing one or more portions of the pledget to an aqueous solution or suspension containing a calcium sugar phosphate. Examples of methods for exposing a tampon pledget to an aqueous solution include, for example spraying the aqueous solution on the pledget, dipping the pledget in the aqueous solution or washing the pledget with the aqueous solution. Alternatively, or in addition, a calcium sugar phosphate can be incorporated in the tampon after compression, such as, for example by exposing a substantially completed tampon to an aqueous solution containing the calcium and then drying the tampon.

While the distribution of the calcium sugar phosphate on or within a hygiene article of the present invention, such as a tampon, can vary as needed, in certain embodiments, the calcium sugar phosphate present in the one or more portions of the hygiene article can be distributed such that suitable effectiveness for reducing or prohibiting the production of TSST-1 on or within the hygiene article can be attained. The calcium sugar phosphate included in the one or more portions of a hygiene article of the present invention can be fugitive, loosely adhered, bound, partially bound, substantially bound, or any combination thereof.

A hygiene article of the present invention can optionally include other beneficial components commonly found in pharmaceutical compositions, such as, for example vitamins, herbs, aloe, moisturizers, botanicals, supplementary antimicrobials, anti-parasitic agents, antipruritics, astringents, local anesthetics, or anti-inflammatory agents. In certain embodiments, the calcium can work in conjunction with one or more of the optionally included components in a complementary or synergistic way.

The present invention is further illustrated by the following examples, which should not be construed as limiting in any way.

### EXAMPLES

### EXAMPLE 1

This example demonstrates the reduction of the amount of TSST-1, as measured by the Enzyme-Linked ImmunoSorbent Assay (ELISA), produced by *S. aureus* grown using the shake flask assay, upon the addition of low concentrations, less than about 5 mM Ca, of calcium sugar phosphates of the present invention.

### Materials and Methods

### Shake Flask Method

The Shake Flask Method was performed in triplicate with appropriate controls. Twenty-five mL of Brain Heart Infusion broth (BHI) (Difco-BD Biosciences, Bedford, MA) was dispensed into 250 mL flasks and the flasks were covered. The medium was autoclaved at 121°-124°C for 15 minutes and allowed to cool to room temperature.

A calcium sugar phosphate solution was prepared by dissolving an appropriate amount of calcium sugar phosphate into 2.5 mM Phosphate Buffered Saline (PBS)/150 mM sodium chloride, pH 7.0. The calcium sugar phosphate solution was added to the BHI medium supplemented with 1% yeast extract at a volume ratio of 25mL:25mL. Each dilution was tested in triplicate. Each dilution was added to a 250mL Erlenmeyer flask. The flasks were inoculated with approximately 10⁶ CFU/mL of an 18 - 24 hour culture of *S. aureus* MN 8 then incubated at 37°C in ambient air supplemented with 5% CO₂ while shaking for 18 - 20 hours. Culture fluid was streaked onto a Blood Agar Plate to ensure purity. A corresponding control of PBS and BHI was also tested. The flasks were removed from the shaker and a 3-4mL sample of fluid was aseptically removed. A standard plate count analysis and ELISA were performed using standard techniques. Results for the test solutions were compared to the appropriate control.

### Enzyme-Linked ImmunoSorbent Assay (ELISA)

*Purification of TSST-1: S. aureus* MN 8 was grown using the shake flask method, as described above. Supernatant from the sample was then subjected to alcohol precipitation, flatbed isoelectric focusing, and gel chromatography. It was determined that the purified TSST-1 preparation had a molecular weight average of 23kD as determined by sodium dodecyl sulfate-polyacrylamide gel electrophoresis, and an isoelectric point of 7.2 as determined by analytical isoelectric focusing. Purity was ascertained by polyacrylamide gel electrophoresis and serological analyses.

*Antibody to TSST-1:* Antisera were obtained by immunization of New Zealand White rabbits. TSST-1 was administered subcutaneously with complete Freund adjuvant on a weekly basis, starting at 10 µg and increasing to 50 µg by increments of 10 µg per week. Rabbits were tested for antibody 2 weeks after the last immunization and were bled if a high antibody titer to TSST-1 was demonstrable by double immunodiffusion. IgG was further prepared for use in the ELISA by precipitation of the serum three times with 35% ammonium sulfate and stored in PBS at - 20°C.

*ELISA reagents:* Carbonate-bicarbonate buffer, pH 9.6, was used as the coating buffer. The diluent and washing solution was PBS, pH 7.4, containing 0.05% Tween 20 and 0.1% bovine serum albumin (PBS-Tween). The substrate solution was p-nitrophenyl phosphate (no. 104: Sigma Chemical Co., St. Louis, Mo.) (1 mg/mL) in 10% diethanolamine buffer, pH 9.8. All solutions contained 0.02% sodium azide.

*TSST-1-enzyme conjugation:* TSST-1 was conjugated to alkaline phosphatase (type VII-S: Sigma). A 100-ul volume of TSST-1 (10 mg/mL in PBS) was mixed with a 600-µl sample of alkaline phosphatase suspended in 3.2 M (NH₄)₂SO₄. After dialysis against PBS, 20 µl of 4% glutaraldehyde was added: the solution was mixed for 2 h, brought up to 2 mL with PBS, and again dialyzed against PBS. Insoluble material was eliminated by centrifugation.

TSST-1 conjugated to alkaline phosphatase was separated from unconjugated toxin and enzyme by gel chromatography over a column (2.6 by 40 cm) of Ultragel AcA 44 (LKB Instruments. Inc., Gaithersburg, Md.). Fractions (1 mL) were collected and analyzed for alkaline phosphatase activity and immunoreactivity. Alkaline phosphatase was detected by mixing 10 µl of 1:10 dilution of each fraction with 3.0 mL of substrate solution. Tubes were read by spectrophotometry at a wavelength of 405 nm after 5 to 10 min. at the same rate at which diluted fractions had been added. Immunoreactivity was determined with a microtiter plate previously coated with antibody to TSST-1 (see below). A 50-µl portion of each column fraction, diluted 1:4 in PBS-Tween, was added to a well and the plate was incubated overnight. Wells were washed, substrate solution was added, and color development was read at 405 nm on a microplate reader (model EL 307. Bio-tek Instruments. Burlington, Vt.). Fractions that contained alkaline phosphatase and were immunoreactive were pooled: bovine serum albumin and sodium azide were added at concentrations of 1.0 and 0.1%, respectively, and the conjugate was stored at 4°C. Conjugate thus prepared showed no evidence of decreased activity at 1 month.

*Immunoassay:* Nonperimeter wells of flat-bottomed, polystyrene microtiter plates (Immulon-2: Dynatech Laboratories, Inc. Alexandria, VA.) were coated with diluted TSST-1 antibody by incubating 200 µl of solution in all wells for 4 h at 3°C. Plates were then washed three times with PBS-Tween. Plates coated in batches were washed again with distilled water, dried over calcium sulfate, covered with plate sealers (Dynatech), and stored at 4°C until use. Plates prepared in this manner were stable for at least 1 month. The optimal dilution of coating antibody was determined by checkerboard titration with TSST-1 conjugate. Final concentrations of both reagents were chosen to yield an optimal density of 1.0 at 60 min after the addition of substrate solution.

Culture supernatants to be assayed were diluted in PBS-Tween. At least three dilutions of each supernatant were tested to assure that the TSST-1 concentration would fall within the range of the standard curve. Samples (100 µl) of each dilution were added to coated wells in duplicate, followed by 100 µl of appropriately diluted TSST-1 conjugate. A standard curve was run in duplicate on each plate with unlabeled TSST-1 at concentrations indicated below. After overnight incubation at room temperature in a humidified box, wells were again rinsed three times with PBS-Tween: 200 µl of prewarmed substrate solution was added, and the plate was incubated at 37°C. Color development was quantitated by spectrophotometry at 405 nm on the microplate reader when wells containing uninhibited TSST-1 conjugate reached an optical density of 1.0, usually after about 60 min.

A standard curve for each experiment was established by plotting optical density as a function of the logarithm of TSST-1 concentration. The correlation coefficient of the linear portion of the curve was derived on a programmable calculator. For sample dilutions with optical densities within the linear portion of the curve, the log of TSST-1 concentration was derived from the mean of duplicate optical density readings. Taking the antilog and correcting for same dilution provided the TSST-1 concentration in the culture supernatant.

TSST-1 concentrations are expressed in micrograms per milliliter to the nearest decimal place. Reproducibility is expressed in terms of the 95% confidence intervals of the mean, based upon the standard deviation (SD) of the log of TSST-1 concentrations (95% confidence interval = mean = 1.96 (SD/√n)].

The above described ELISA methodology is described in the journal article, Competitive, Enzyme-Linked Immunosorbent Assay for Toxic Shock Syndrome Toxin 1; Journal of Clinical Microbiology, July 1985, Vol. 22 No. 1, p. 26-31.

**TABLE 1**

| Compound | Ca (mM) added to shake flask | % TSST-1 Change (µg/ml) vs. control | % TSST-1 Change (µg/ml/mM) vs. control |
|---|---|---|---|
| Comparative Examples (Calcium Salts) | | | |
| calcium chloride | 15.1 | 37 | 2.5 |
| calcium stearate* | 9.9 | 65 | 6.6 |
| calcium lactate | 8.8 | 49 | 5.6 |
| calcium citrate malate | 6.8 | 89 | 13.1 |
| | | | |
| calcium glycerophosphate | 3.6 | 53 | 14.7 |

| | | | |
|---|---|---|---|
| *calcium stearate solid is added directly into the media | | | |

This example demonstrates that a calcium sugar phosphate, in this instance calcium glycerophosphate, reduced *S. aureus* TSST-1 production by about 53%, as compared to the control of PBS and BHI.

### EXAMPLE 2

This example demonstrates the ability of calcium sugar phosphates to almost completely disrupt TSST-1 production at higher concentrations. In addition, the example demonstrates that at higher concentrations calcium sugar phosphates are more effective at reducing TSST-1 production than calcium salts.

### Materials and Methods

The materials and methods used *(Shake Flask Method and ELISA)* were the same as those used for EXAMPLE 1.

**TABLE 2**

| Compound | Ca (mM) added to shake flask | % TSST-1 Change (µg/ml) vs. control | % TSST-1 Change (µg/ml/mM) vs. control | Solubility (mM of Calcium in H₂O at 25°C) |
|---|---|---|---|---|
| Comparative Examples (Calcium Salts) | | | | |
| calcium chloride | 75.6 | 82 | 1.1 | 7324 |
| calcium stearate* | 98.8 | 62 | 0.6 | <0.003 |
| calcium lactate | 53.1 | 89 | 1.7 | 229 |
| calcium citrate malate | 22.6 | 88 | 3.9 | 45.1 |
| | | | | |
| calcium glycerophosphate | 36.0 | 98 | 2.7 | 95.2 |

| | | | | |
|---|---|---|---|---|
| *calcium stearate solid is added directly into the media | | | | |

This example demonstrates that calcium sugar phosphates, in this instance calcium glycerophosphate: (1) at higher concentrations of about 36.0 mM Ca (as compared to the concentrations of Table 1 of about 3.6 mM) almost completely disrupt TSST-1 production with about a 98% reduction of TSST-1 production, as compared to the control of PBS and BHI; (2) reduces TSST-1 production to a greater degree (about 98%) as compared to other calcium salts (calcium chloride-82%, calcium stearate-62%, calcium lactate-89%, calcium citrate malate-88%). It is believed that sugar phosphate played an important role in the greater efficiency in reducing TSST-1 production than other anions for example, stearate, lactate and citrate malate; (3) has a greater solubility (95.2 mM) than calcium citrate malate (45.1 mM) and calcium stearate (<0.003 mM), it is believed the higher solubility of calcium glycerophosphate may also contribute to the greater degree of TSST-1 reduction (about 98%) as compared to the TSST-1 reduction of calcium citrate malate (88%) and calcium stearate (62%) whose TSST-1 reduction levels remained about the same, as compared to their TSST-1 reduction at lower concentrations as shown in Table 1 (calcium citrate malate at 6.8 mM Ca reduced TSST-1 production by 89% and at 22.6 mM Ca reduced TSST-1 production by 88% -and- calcium stearate at 9.9 mM Ca reduced TSST-1 production by 65% and at 98.8 mM Ca reduced TSST-1 production by 62%).

### EXAMPLE 3

This example demonstrates that calcium sugar phosphates are more effective at reducing TSST-1 production by *S. aureus* than sodium sugar phosphates.

### Materials and Methods

The materials and methods used *(Shake Flask Method and ELISA)* were the same as those used for EXAMPLE 1.

**TABLE 3**

| Compound | Anion (mM) added to shake flask | % TSST-1 Change (µg/ml) vs. control |
|---|---|---|
| sodium glycerophosphate | 38.9 | 31 % reduction |
| calcium glycerophosphate | 36.0 | 98% reduction |

This example demonstrates that calcium sugar phosphates, in this instance calcium glycerophosphate, are more effective at reducing TSST-1 production than sodium sugar phosphates, in this instance sodium glycerophosphate, as demonstrated in Table 3, wherein calcium glycerophosphate at a concentration of 36.0 mM reduced TSST-1 production by 98%, as compared to sodium glycerophosphate at a concentration of 38.9 mM which reduced TSST-1 production by only 31%.

### EXAMPLE 4

This example shows that calcium sugar phosphates have the ability to almost completely disrupt TSST-1 production while being substantially non-lethal to *S. aureus.*

### Materials and Methods

The materials and methods used *(Shake Flask Method and ELISA)* were the same as those used for EXAMPLE 1.

**TABLE 4**

| Compound | Ca (mM/mL) Concentration added to shake flask | % TSST-1 Change (µg/ml) vs. control | Change in *S*. *aureus* cfu/mL vs. control |
|---|---|---|---|
| calcium glycerophosphate | 36.0 | 98 | <2 log |

This example demonstrates that calcium sugar phosphates such as calcium glycerophosphate are able to reduce TSST-1 production while being substantially non-lethal to *S*. *aureus.*

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A hygiene article comprising calcium sugar phosphate, wherein the calcium sugar phosphate reduces *S. aureus* TSST-1 production as measured by ELISA, as compared to TSST-1 production by *S. aureus* to which no calcium sugar phosphate has been added; wherein the hygiene article is a feminine article; and wherein the feminine article is at least one of a tampon, sanitary napkin, intralabial device, incontinence article, liner, cervical cap, contraceptive sponge, menstrual cup, or contraceptive diaphragm.

2. The hygiene article of claim 1, wherein the calcium sugar phosphate comprises: 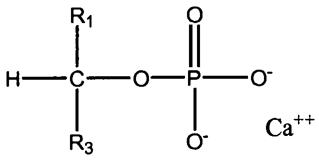 Wherein:
R₁, R₃ = H; (CHOH)ₙCH₂OH; (CHOH)ₙC(O)R₄; (CHOH)ₙ CHO-PO₃²⁻
R₄ = H; (CHOH)ₘ CH₂OH; (CHOH)ₘ CHO-PO₃²⁻
n =0- 5
m = 0-4
n+m =0-4

3. The hygiene article of claim 1 or 2, wherein the calcium sugar phosphate is at least one of a calcium dihydroxyacetone phosphate, calcium glycerophosphate, calcium hexosephosphate, calcium inositol phosphate, calcium mannitol phosphate, or calcium pentosephosphate.

4. The hygiene article of any of the preceding claims, wherein the calcium sugar phosphate is substantially non-lethal to *S*. *aureus* when measured by the Shake Flask Method.

5. The hygiene article of any of the preceding claims, wherein the calcium sugar phosphate has a solubility that provides a Ca concentration in H₂O of at least about 50mM at 25°C.

6. The hygiene article of any of the preceding claims, wherein the feminine article is a tampon comprising absorbent foam.

## Patentansprüche

1. Hygieneartikel, umfassend Calciumzuckerphosphat, wobei das Calciumzuckerphosphat die Produktion von S. aureus-TSST-1 gemäß Messung durch ELISA im Vergleich zur TSST-1-Produktion durch S. aureus, zu dem kein Calciumzuckerphosphat hinzugegeben wurde, reduziert; wobei der Hygieneartikel ein Damenartikel ist; und wobei der Damenartikel mindestens ein Artikel aus Tampon, Damenbinde, intralabialer Vorrichtung, Inkontinenzartikel, Einlage, Portiokappe, empfängnisverhütendem Schwamm, Menstruationstasse oder empfängnisverhütendem Diaphragma ist.

2. Hygieneartikel nach Anspruch 1, wobei das Calciumzuckerphosphat umfasst: 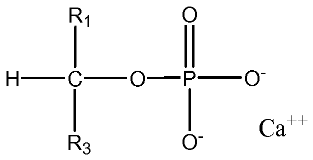 worin:
R₁, R₃ = H; (CHOH)ₙCH₂OH; (CHOH)ₙC(O)R₄; (CHOH)ₙ CHO-PO₃²⁻
R₄ = H; (CHOH)ₘ CH₂OH; (CHOH)ₘ CHO-PO₃²⁻
n = 0-5
m = 0-4
n+m = 0-4

3. Hygieneartikel nach Anspruch 1 oder 2, wobei das Calciumzuckerphosphat mindestens eines von Calciumdihydroxyacetonphosphat, Calciumglycerophosphat, Calciumhexosephosphat, Calciuminositphosphat, Calciummannitphosphat oder Calciumpentosephosphat ist.

4. Hygieneartikel nach einem der vorstehenden Ansprüche, wobei das Calciumzuckerphosphat im Wesentlichen nicht letal für S. aureus bei Messung durch das Schüttelkolbenverfahren ist.

5. Hygieneartikel nach einem der vorstehenden Ansprüche, wobei das Calciumzuckerphosphat eine Löslichkeit besitzt, die eine Ca-Konzentration in H₂O von mindestens etwa 50 mM bei 25 °C liefert.

6. Hygieneartikel nach einem der vorstehenden Ansprüche, wobei der Damenartikel ein Tampon ist, das einen Absorptionsschaum umfasst.

## Revendications

1. Article d'hygiène comprenant du phosphate de sucre de calcium, dans lequel le phosphate de sucre de calcium réduit la production de TSST-1 par S. aureus mesurée par test ELISA, comparée à la production de TSST-1 par S. aureus sans addition de phosphate de sucre de calcium ; l'article d'hygiène étant un article féminin ; et l'article féminin étant au moins un tampon, une serviette hygiénique, un dispositif intralabial, un article pour l'incontinence, une doublure, une coiffe cervicale, une éponge contraceptive, une coupelle menstruelle ou un diaphragme contraceptif.

2. Article d'hygiène selon la revendication 1, dans lequel le phosphate de sucre de calcium est constitué par : 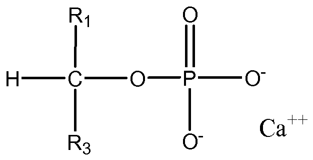 dans laquelle :
R₁, R₃ = H ; (CHOH)ₙCH₂OH ; (CHOH) ₙC(O)R₄ ; (CHOH)ₙCHO-PO₃²-
R₄ = H ; (CHOH)ₘCH₂OH ; (CHOH)ₘCHO-PO₃²-
n =0-5
m = 0-4
n+m =0-4

3. Article d'hygiène selon la revendication 1 ou 2, dans lequel le phosphate de sucre de calcium est au moins une dihydroxyacétone phosphate de calcium, un glycérophosphate de calcium , un hexosephosphate de calcium, un inositol phosphate de calcium, un mannitol phosphate de calcium, ou un pentosephosphate de calcium.

4. Article d'hygiène selon l'une quelconque des revendications précédentes, dans lequel le phosphate de sucre de calcium est essentiellement non létal pour S. aureus en mesurant par la méthode par agitation en flacon.

5. Article d'hygiène selon l'une quelconque des revendications précédentes, dans lequel le phosphate de sucre de calcium a une solubilité qui permet une concentration de Ca dans H₂O d'au moins environ 50 mM à 25 oC.

6. Article d'hygiène selon l'une quelconque des revendications précédentes, l'article féminin étant un tampon contenant de la mousse absorbante.
